# EUROPEAN PATENT SPECIFICATION

(11) **EP 1 865 326 B1**
(45) Date of publication and mention of the grant of the patent: **07.09.2011**
(21) Application number: 06011829.6
(22) Date of filing: 08.06.2006
(51) Int. Cl.: G01N 33/68

(54) **Assay for the diagnosis of Alzheimer's Disease based on the determination of the ratio of secretase Aß cleavage products**
Assay zur Diagnose von Alzheimer basierend auf der Bestimmung des Verhältnisses von Sekretase-Aß-Spaltprodukten
Analyse de diagnostic de la maladie d'Alzheimer basée sur la détermination du rapport de produits de division secrétase Aß

(43) Date of publication of application: 12.12.2007
(73) Proprietor: FU Berlin, 14195 Berlin (DE)
(72) Inventor: Multhaup, Gerd, Prof. Dr., 14532 Kleinmachnow (DE); Münter, Lisa, 10437 Berlin (DE)
(74) Representative: Schüssler, Andrea

(56) References cited:
- WO-A-02/02769
- WO-A-98/21589
- WO-A-03/103593
- WO-A2-00/17369
- WO-A2-03/001881
- WO-A2-03/040183
- WO-A2-2005/116640
- WILTFANG J ET AL: "Highly conserved and disease-specific patterns of carboxyterminally truncated Abeta peptides 1-37/38/39 in addition to 1-40/42 in Alzheimer's disease and in patients with chronic neuroinflammation" JOURNAL OF NEUROCHEMISTRY, vol. 81, no. 3, May 2002 (2002-05), pages 481-496, XP002397538 ISSN: 0022-3042
- BLENNOW ET AL: "Cerebrospinal Fluid Protein Biomarkers for Alzheimer's Disease" JOURNAL OF THE AMERICAN SOCIETY FOR EXPERIMENTAL NEUROTHERAPEUTICS, XX, XX, vol. 1, no. 2, April 2004 (2004-04), pages 213-225, XP005321033 ISSN: 1545-5343
- ANDREASEN N ET AL: "CSF biomarkers for mild cognitive impairment and early Alzheimer's disease" CLINICAL NEUROLOGY AND NEUROSURGERY, ELSEVIER, AMSTERDAM, NL, vol. 107, no. 3, April 2005 (2005-04), pages 165-173, XP004917240 ISSN: 0303-8467
- GOLDE T E ET AL: "Biochemical detection of Abeta isoforms: implications for pathogenesis, diagnosis, and treatment of Alzheimer's disease" BIOCHIMICA ET BIOPHYSICA ACTA. MOLECULAR BASIS OF DISEASE, AMSTERDAM, NL, vol. 1502, no. 1, 26 July 2000 (2000-07-26), pages 172-187, XP004276984 ISSN: 0925-4439
- WILTFANG J ET AL: "Highly conserved and disease-specific patterns of carboxyterminally truncated Abeta peptides 1-37/38/39 in addition to 1-40/42 in Alzheimer's disease and in patients with chronic neuroinflammation" JOURNAL OF NEUROCHEMISTRY, vol. 81, no. 3, May 2002 (2002-05), pages 481-496, XP002398797 ISSN: 0022-3042
- HILBUSH ET AL: "New Prospects and Strategies for Drug Target Discovery in Neurodegenerative Disorders" JOURNAL OF THE AMERICAN SOCIETY FOR EXPERIMENTAL NEUROTHERAPEUTICS, XX, XX, vol. 2, no. 4, October 2005 (2005-10), pages 627-637, XP005321006 ISSN: 1545-5343
- CHANG L ET AL: "FEMTOMOLE IMMUNODETECTION OF SYNTHETIC AND ENDOGENOUS AMYLOID-BETA OLIGOMERS AND ITS APPLICATION TO ALZHEIMER'S DISEASE DRUG CANDIDATE SCREENING" JOURNAL OF MOLECULAR NEUROSCIENCE, BIRKHAEUSER, CAMBRIDGE, MA, US, vol. 20, no. 3, 2003, pages 305-313, XP008062752 ISSN: 0895-8696

## Description

The present invention relates to a method for the diagnosis of Alzheimer's disease (AD) or a particular stage of the disease which is based on the determination of the ratio of at least two y-secretase cleavage products, Aβ48, Aβ45, Aβ42, Aβ38 and Aβ35, wherein at least the ratio of Aβ38:Aβ42 is determined. A decreased ratio of Aβ38:Aβ42 as compared to the normal ratio is indicative of AD.

For the diagnosis of Alzheimer's disease (AD) and for distinguishing AD from other dementia various methods are used. These diagnostic methods comprise the "scanning" of the brain using computer tomography (CT), magnetic resonance imaging (MRI) or positron emission tomography (PET) in order to visualize abnormalities in the brain. Another diagnostic approach is the mini-mental state examination (MMSE) which is based on the testing of particular cognitive problems (short-term memory, long-term memory, repetition tests), thus, allowing a distinct evaluation of the patient's capability as regards resolving of problems, linguistic competence etc. According to neurologists, geriatricians and psychiatrists who are familiar with a variety of dementia this test allows a precise diagnosis. Thus, MMSE forms the basis of current diagnosis.

An additional diagnostic method is based on the determination of compounds which are characteristic of the disease (biochemical markers, biomarkers) in body fluids like blood, liquor, urine etc. using selective laboratory tests. This approach allows the determination of changes of the concentrations due to the disease - even early in the course of the disease, when clinical symptoms might be mild and vague. However, this assay is problematic, since low concentrations of biochemical markers have to be determined selectively for early diagnosis and for exactly distinguishing between AD and other dementia. In order to enhance diagnostic accuracy, particular biochemical markers like the β-amyloid protein and the tau protein are used. The cerebrospinal fluid (CSF) level of tau has been suggested to reflect neuronal and axonal degeneration or possibly formation of neurofibrillary tangles. Accordingly, tau is regarded as a general biomarker for a neuronal dysfunction (Creutzfeldt-Jacob, AD etc.). Tau levels moderately increase in AD (there is a stronger increase in CID) and in other neurodegenerative diseases whereas phosphor-tau strongly increases in AD.

Currently, amyloid β (Aβ) is regarded as the most important diagnostic parameter, since this peptide is responsible for the plaque like extra cellular depositions (amyloidosis) in the brain which is a characteristic feature early in the course of AD. APP is a type 1 transmembrane (TM) protein, which undergoes proteolytic processing by several secretases. First, the bulk of the ectodomain needs to be removed by membrane-bound α- or β-secretases leading to secreted forms of APP and membrane bound C-terminal fragments α-CTF or β-CTF, respectively. Regulated intramembrane proteolysis (RIP) of the β-CTF by γ-secretase occurs only after ectodomain shedding and releases the Aβ peptide from the membrane. The active γ-secretase complex consists of the four subunits presenilin-1 (PS-1), APH-1, PEN-2 and nicastrin. PS-1 contains two catalytic active aspartate residues in TMS-6 and TMS-7. PS-1 is endoproteolytically cleaved and the N-terminal and C-terminal fragments were shown to exist as dimers in the catalytic core of γ-secretase. The amino termini of CTF stubs are recognized by nicastrin, which functions as a γ-secretase substrate receptor. The γ-secretase cleaves at variable sites thus generating Aβ peptides of varying lengths. Aβ peptides are closely linked to AD as they accumulate to amyloid plaques. Aβ42 is the pathologically most relevant form of the Aβ species since it is more prone to aggregation and found elevated in AD patient brains.

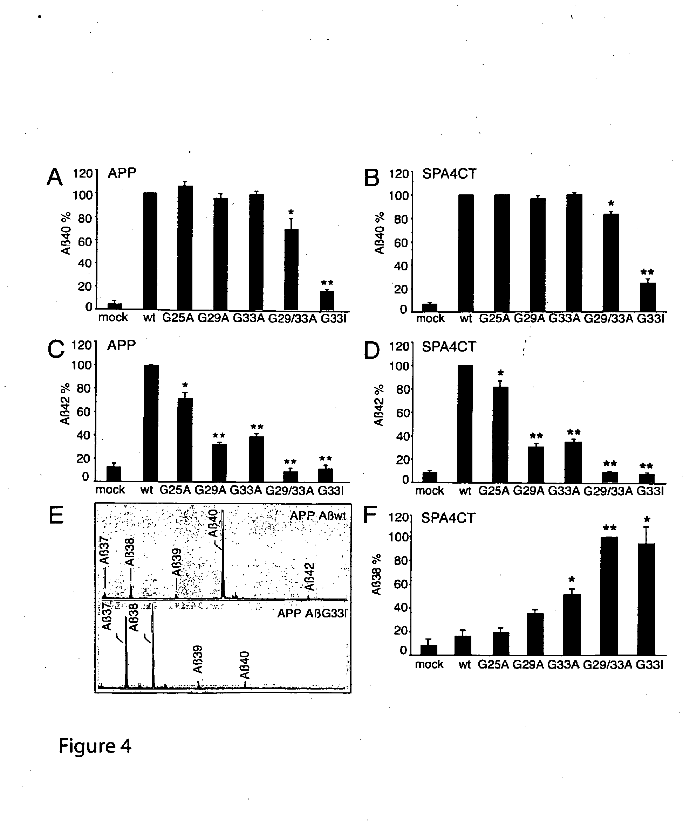

A recently discussed mechanism of y-secretase cleavage implies sequential proteolytic cleavage steps to release Aβ. Accordingly, the first cut occurs at the cytoplasmic edge of the TMS at the ε-site, i.e. residue 49 of β-CTF. The product Aβ49 remains membrane-bound and is further processed in a sequential action mode into Aβ46 (ζ-site) and Aβ40 or Aβ42 (γ-site) (Figure 4A). Notch was recently reported to be processed by y-secretase by a similar sequential mechanism. Previously it was shown that APP forms homodimers as it has been observed for other γ-secretase substrates, e.g. the receptor tyrosine-protein kinase ErbB-4 and E-cadherin. The APP homodimerization is likely to be mediated by two different sites of the ectodomain, the loop region encompassing residues 91-111 and a second site overlapping with the collagen binding site spanning residues 448-465.

In a recent study, the levels of Aβ40 and Aβ42 of AD patients and of healthy individuals were compared. It could be shown that there were no differences in both groups as regards the levels of Aβ40, however, AD patients showed decreased levels of Aβ42. Unfortunately, the absolute concentrations of Aβ42 observed in various AD patients differ dramatically ("high" individuals vs. "low" individuals). These variations of values of absolute concentrations show that the data obtained have been affected by various factors (state of the sample, assay system used, etc.). Accordingly, the establishing of limit values which is required for the development of a reliable diagnostic assay is almost impossible. Moreover, in the course of sporadic AD disease various levels of amyloid could be observed. Accordingly, the determination of the concentration of Aβ42 only is not sufficient for precise diagnosis of AD. In other words, at present, there is no reliable marker available for diagnosing AD, a particular stage of AD or for diagnosing an elevated risk for developing AD.

### (insertion page 3a)

Thus, the technical problem underlying the present invention is to provide a marker for the reliable (early) diagnosis of Alzheimer's disease (AD) or a particular stage of said disease.

Wiltfang et al., J. of Neurochemistry, 81 (2002), p. 481-496 describe highly conserved and disease-specific patterns of carboxyterminally truncated Aß peptides 1-37/38/39 in addition to 1-40/42 in Alzheimer's disease and in patients with chronic neuroinflammation.

Blennow, K., NeuroRx, Vol. 1 (2004), pp. 213-225 describe cerebrospinal fluid protein biomarkers for Alzheimer's disease.

Andreasen et al., Clinical Neurology and Neurosurgery, 107 (2005), pp. 165-173 describe CSF biomarkers for mild cognitive impairment and early Alzheimer's disease.

Golde et al., Biochemica and Biophysica Acta 1502 (2000), pp. 172-187 is a review article about the biochemical detection of Aß isoforms.

The solution to said technical problem is achieved by providing the embodiments characterized in the claims. During the experiments leading to the present invention a GxxxG motif could be identified mediating helix-helix interactions within the transmembrane segment of APP. This motif represents a unique homophilic interaction site of the γ-secretase substrate CTF and controls the processing into Aβ via the final γ-secretase cleavages. Mutations of this motif and subsequent analysis of Aβ species revealed that consecutive cuts occur by the two sets of cleavages identified, (I) 49-46-43-40-37-34 and (II) 48-45-42-38-35 (Aβ numbering). There is increasing evidence that these species are generated by consecutive helix-turn by helix-turn cleavages by the γ-secretase starting from the C-terminus and progressing to the N-terminus of the substrate of β-CTF. These findings have a direct impact on diagnostic approaches that monitor the progress of the disease. Accordingly, the ratio of Aβ38/Aβ42 should be an ideal marker not only for a specific diagnosis of AD but also to determine the exact stage of the disease. In addition, it was found that the ratio of Aβ38/Aβ37 from two product lines could enhance the reliability of existing test systems by including a second reading point of the two product lines of γ-secretase activity.

Based on a FRET approach, the results of the present study moreover provide compelling evidence that APP forms dimers in living cells. Considering that APP homodimers are formed as early as in the ER, the two ectodomain contact sites may initiate the interaction. The two captured polypeptide chains then align and dock in the lipid bilayer where they are further stabilized by consecutive GxxxG motifs of the TMS of APP. This motif provides a template for dimerization of two α-helices in a hydrophobic environment. Similar to APP, other γ-secretase substrates, which have a single GxxxG motif in their TMS were described to form full-length homodimers, such as ErbB-4, E-cadherin, Nectin-1 and APLP-1.

The present work also shows that this interaction is primarily mediated by the glycine residues G29 and G33 of the GxxxG motifs and establishes a crucial functional role of the GxxxG motifs in APP processing. It could be demonstrated that dimeric (wt) as well as mainly monomeric substrates, i.e. β-CTF mutant G33I, are equally well cleaved by γ-secretase. For GxxxG mutants an inverse shift in Aβ42 and Aβ38 generation was observed while the Aβ40 level was specifically diminished for the dimerization-disrupting mutant G33I.

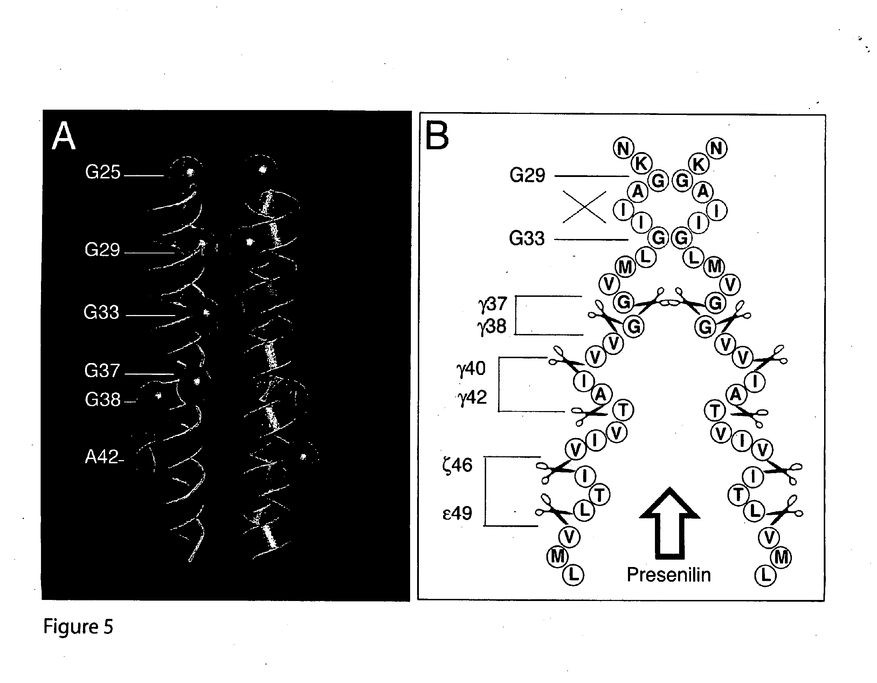

Based on a recently presented model, γ-secretase processing is a continuous process starting from the C-terminal end of the TMS at position 49/48 followed by cleavages at position 46/45 and 42/40 thus removing helix turn by helix turn. Under normal conditions processing of β-CTF is largely arrested at the step 42/40 leading to high levels of Aβ42 and Aβ40. According to the present data the sequential cleavage model is expanded by proposing that this arrest is due to the dimeric state of the substrate β-CTF and that the two α-helices of the dimeric substrate form a cross-point at residues G29 and G33 leading to a sterical hindrance for γ-secretase (Figure 5B). APP TMS dimerization is unique insofar as it is the only known γ-secretase substrate with a GxxxG motif in triplicate at the start of the TMS. Any variations in APP TMS dimerization strength may fine-tune the actual preferred cleavage site by suspending the sterical hindrance. Candidate factors are non-steroidal anti-inflammatory drugs (NSAIDs) that are reported to affect the generation of different Aβ species like sulindac sulfide and flurbiprofen. Similar to the GxxxG mutants, depending on the NSAID tested they reduce Aβ42 levels and selectively increase Aβ38 production or vice versa. For most NSAIDs the Aβ40 levels also remain unaffected. Thus, some NSAIDs might directly affect the dimerization strength of the substrate β-CTF. However, any effects of NSAIDs on GxxxG motifs of TMS-7 of PS-1 or TMS-4 or TMS-6 of APH-1 altering the active site conformation might also be possible.

Reduced dimerization strength of β-CTF allows γ-secretase to further proceed with cleavages to N-terminal positions generating Aβ38 and Aβ37 (Figure 5B). Therefore, the present results confirm and expand the recently presented model of the γ-secretase cleavage model providing a detailed insight into the principal cleavage mechanism of this enzyme (Figure 5B). In case of APP, dimerization of the TMS through the residues G29 and G33 of the GxxxG motifs determines where the final γ-secretase cleavage site might occur and if Aβ42 can leave the membrane or shorter forms are produced. This advanced model would predict that γ-secretase is not only involved in AICD production but has also a function in Aβ-clearance by cleaving off turn by turn until more hydrophilic forms of Aβ are released from the membrane, such as Aβ38.

In summary, according to the experimental observations in the present study two different product lines of γ-secretase cleavage activities exist, i.e., **(I) 49-46-43-40-37-34** and **(II) 48-45-42-38-35.** These species are generated by independent cleavages of the γ-secretase starting from the C-terminus and progressing to the N-terminus of the substrate β-CTF. In AD the levels of products of the second product line are varying whereas the product levels of the first line (I) remain constant. Thus, any ratios between γ-secretase cleavage products of product line II will provide information on the disease and the stage of the disease. Since levels of the species 42 and 38 are most prominent compared to others of the same line and γ-secretase is modulated by dimerization of the substrate such that Aβ42 increases in early disease states, it has been decided to take the ratio Aβ38/Aβ42 as a marker.

Finally, it can be concluded that a strong dimerization of the substrate β-CTF is central to AD since any events stabilizing dimerization would increase the production of Aβ42 that can easily form toxic oligomers. Compounds targeting the dimerization of the APP TMS and interfering with the G₂₉xxxG₃₃ interaction motif of two y-secretase substrate molecules may be useful as therapeutics to prevent generation of Aβ42.

### BRIEF DESCRIPTION OF THE DRAWINGS

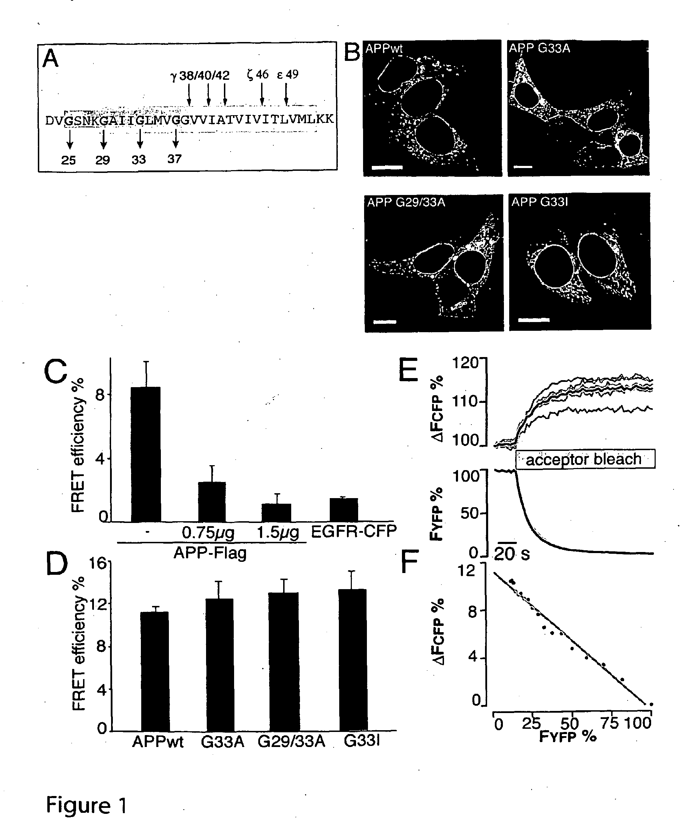

Figure 1: APP full-length dimerization is independent of the GxxxG motifs
   **(A)** APP-TMS sequence. Glycine residues of the three consecutive GxxxG motifs are numbered (grey box). The membrane embedded sequence is marked with a yellow box. γ-secretase cleavage sites are indicated above the sequence by arrows.
   **(B)** Confocal microscopy images of APPwt-YFP and APP-YFP mutants show equal sub cellular localization; bars: 10 µm.
   **(C)** FRET approach: Competition of fluorescent APPwt dimers with APPwt-Flag (means ± s.e.m., n = 4 with 3-6 cells each). As a negative control, APP-YFP wt does not interact with EGFR-CFP.
   **(D)** FRET efficiencies of wt and mutant APP (means ± s.e.m., n = 5 evaluated in quadruplicate). Note that APPwt FRET efficiency in C is lower compared to D due to lower amounts of plasmids and thus competition with endogenous APP.
   **(E)** Time course of donor fluorescence recovery (ΔF_{CFP}) during selective photo bleaching of the acceptor (F_{XPP}). Data represent single cells (grey lines) and means (black lines) of a representative cell group.
   **(F)** Linear regression analysis of donor recovery (ΔF_{CFP}) vs. fractional acceptor (YFP) photo bleach. Depicted is the mean of single cells from a representative experiment shown in D.

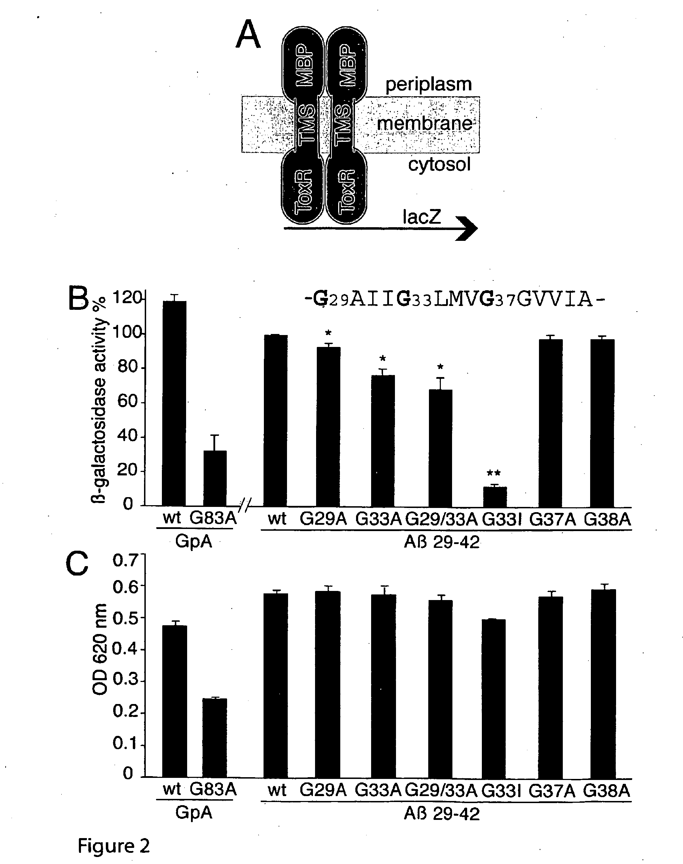

Figure 2: The APP TMS dimerizes via the **G₂₉XXXG₃₃** motif
   **(A)** Representation of ToxR-system chimeric proteins. The TMS under study is fused between the maltose-binding protein (MBP) and the ToxR transcription activator. Upon dimerization, ToxR-domains initiate transcription of the lacZ gene encoding β-galactosidase.
   **(B)** ToxR-assay. The tested Aβ residues 29 to 42wt are indicated. Measured β-galactosidase activity of the wt-sequence was set as 100 % (mean ± s.e.m., n = 4-6). Mutations to alanine at positions G29 and especially G33 reduce dimerization strength. Mutation G29/33A potentates the effects of the single mutants and G33I acts strongly disruptive. Asterisks indicate significant differences to Aβ29-42wt (*p < 0.01, **p < 1x10⁻¹⁵, Student's t-test).
   **(C)** All chimera complement MBP-deficiency of E. coli PD28 cells and allow the cells to grow in minimal media indicating correct membrane integration of chimeric proteins.

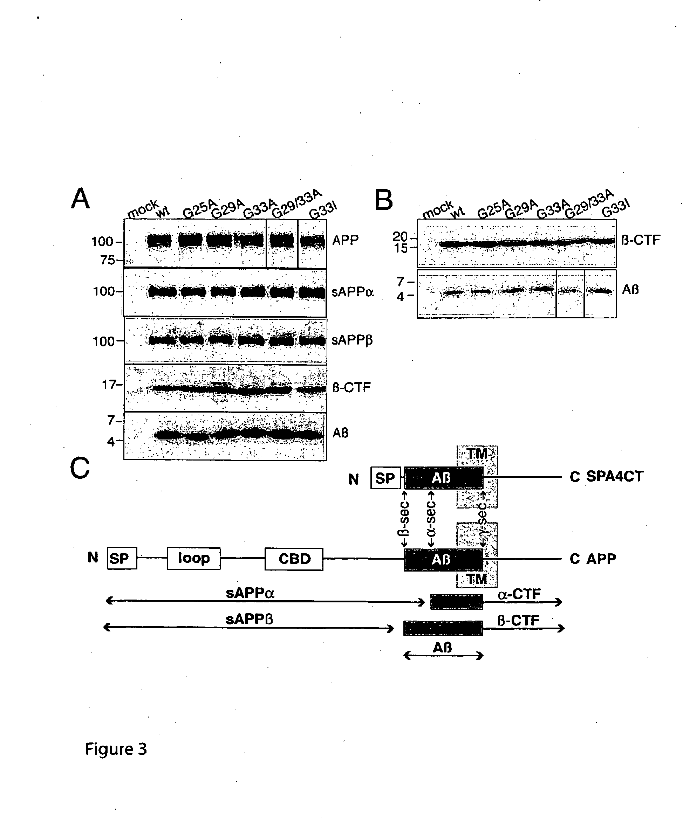

Figure 3: Expression and processing of APP and SPA4CT in stably transfected SHSY5Y cell lines
   **(A)** APP-transfected cells. Antibody 22C11 was used to detect full-length APP (APP). Antibody WO-2 (epitope Aβ residues 2-10) was used to label sAPPα (sAPPα) and to immunoprecipitate C-terminal fragments (β-CTF) and Aβ. Western blot of sAPPβ was probed with antibody 879 (sAPPβ). Vertical bars indicate exchanged lanes for uniform labelling.
   **(B)** SPA4CT-transfected cells. Expression control of immunoprecipitated β-CTF, Western blot probed with antibody WO-2 (β-CTF). Western blot of immunoprecipitated Aβ probed with antibody WO-2 (epitope Aβ residues 2-10).
   **(C)** Schematic representation of the processing of APP and the construct SPA4CT. SP: signal peptide (cleaved off in the endoplasmic reticulum); loop: loop region residues 91111; CBP: Collagen binding site residues 448-465; Aβ: Aβ domain, part of the APP ectodomain and the transmembrane sequence (TM); α- or β-secretases cleave with in the Aβ domain to generate soluble APP (sAPPct or sAPPβ, respectively) and C-terminal fragments (α-CTF or β-CTF, respectively). γ-secretase cleaves β-CTF within the membrane and generates Aβ.
Figure 4: TMS dimerization strength determines the γ-secretase cleavage site and Aβ42 generation
   **(A, C)** Aβ40- and Aβ42-specific ELISAs from media of APP stably transfected SHSY5Y cells and **(B, D)** Aβ40- and Aβ42-specific ELISAs from media of SPA4CT stably transfected SHSY5Y cells.
   **(A-D)** The wt was set as 100% (means ± s.e.m., n = 3-6). Asterisks indicate significant differences to wt (*p < 0.01, ** p < 0.00001, Student's t-test). Dimerization-attenuating mutants do not affect Aβ40 levels but reduce the Aβ42 level significantly. The mutation G33I reduces Aβ40 as well as Aβ42 levels. The mutants G37A and G38A could not be analyzed by ELISA since the mutations affected the epitope of the monoclonal antibodies used to detect Aβ40 or Aβ42.
   **(E)** MALDI-MS spectra of secreted Aβ from APPwt- or APP G33I-transfected cells. The mutation G33I leads mainly to secreted Aβ37 and Aβ38 at the expense of Aβ40 or Aβ42.
   **(F)** Aβ38-specific ELISA from media of SPA4CT stably transfected SH-SY5Y cells, mutant G29/33A was set as 100% (means ± s.e.m., n = 4). Asterisks indicate significant differences to wt (*p < 0.01, ** p < 0.0001, Student's t-test). Aβ38 levels increase when Aβ42 levels decrease of mutants that attenuate dimerization stability.
Figure 5: Model of the APP TMS and γ-secretase cleavage mechanism
   **(A)** Computational molecular model of a low-energy conformation of APP TMS with G29 and G33 in the helix-helix interface.
   **(B)** Postulated γ-secretase cleavage mechanism within the APP TMS. Sequential γ-secretase cleavage proceeds from the carboxyl terminal end to the N terminus (sites as indicated by scissors). The site of the final cleavage is determined by the strength of interaction of the two helices of two APP molecules mediated by the transmembrane residues G29 and G33. The ε- and ζ-cleavages occur independently of the dimeric or monomeric form of the substrate (green scissors). Stable APP-TMS dimers constitute a sterical hindrance for the moving γ-secretase such that the final cleavage occurs after residue 42/40 and produces Aβ42 and Aβ40 (yellow scissors). Only perturbed dimers or monomeric substrates allow the γ-secretase to move an to further N-terminal sites yielding Aβ38 and Aβ37 (red scissors).

The present invention, thus, provides a method for the diagnosis of Alzheimer's Disease (AD) or the stage of AD, said method comprising the following steps:
(a) contacting a sample obtained from a patient with probes specifically binding to at least two γ-secretase cleavage products Aβ48, Aβ45, Aβ42, Aβ38 and Aβ35,; and
(b) determining the amounts of said cleavage products in said sample, wherein at least the Aß38:Aß42 ratio is determined and a decreased ratio of Aß38:Aß42 as compared to a normal ratio is indicative of AD.

The present invention also provides a method for monitoring the progress of an AD therapy, said method comprising the following steps:
(a) contacting a sample obtained from a patient with probes specifically binding to at least two y-secretase cleavage products Aβ48, Aβ45, Aβ42, Aβ38 and Aβ35, ; and
(b) determining the amounts of said cleavage products in said sample, wherein a normalized ratio of said cleavage products as compared to the ratio at the onset of therapy is indicative of a therapeutic effect.

The term "normal ratio" as used herein corresponds to a ratio which can be found in healthy individuals and which is determined by using the average values obtained, e.g., from at least 10, preferably from at least 100 individuals.

Suitable samples for carrying out the diagnostic method of the invention are cerebrospinal fluid (CSF), blood, serum, plasma or urine with CSF and blood being preferred. The sample is taken from the patient using routine methods, e.g. CSF is taken by lumbar puncture.

According to the present invention, the amounts of Aβ38 and Aβ42 in said sample are determined, wherein a decreased ratio of Aβ38:Aβ42 as compared to the normal ratio is indicative of AD.

In more preferred embodiments of the methods of the invention, the amounts of Aβ38 and Aβ42 in said sample are determined, wherein (i) a decreased ratio of Aβ38:Aβ42 as compared to the normal ratio and an increased concentration of Aβ42 as compared to the normal concentration is indicative of an early stage of AD and (ii) a decreased ratio of Aβ38:Aβ42 as compared to the normal ratio and a decreased or normal concentration of Aβ42 as compared to the normal concentration is indicative of a late stage of AD.

In an even more preferred embodiment of the diagnostic method of the present invention a decreased ratio of Aβ38:Aβ42 means Aβ38:Aβ42 ≤ 1.5, preferably ≤ 1.2, more preferably ≤ 1.0.

Any method allowing the determination of the levels of Aβ38, Aβ42 etc. in a sample can be used in the method of the present invention, e.g. immunological assays, size separation, e.g. by gel filtration, amino acid analyses etc.. Immunological assays and MALDI-MS are preferred.

In an even more preferred embodiment of the method of the present invention, the determination of the amounts of Aβ38, Aβ42 etc. is carried out by an immunological assay using antibodies specific for Aβ38 etc., i.e., the anti-Aβ38-antibody does not substantially cross-react with Aβ42 and, vice versa, the anti-Aβ42-antibody does not substantially cross-react with hAβ38. Suitable antibodies are commercially available, e.g. the antibodies G2-10 (anti-Aβ40) and G2-13 (anti-Aβ42) from TGC, Zürich.

The term "antibody" as used herein, preferably, relates to antibodies which consist essentially of pooled monoclonal antibodies with different epitopic specificities, as well as distinct monoclonal antibody preparations. Monoclonal antibodies are made from an antigen containing fragments of the hAβ42 peptide and hAβ38, respectively, by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody.

In a further preferred embodiment of the method of the present invention, the anti-Aβ38-antibody is directed to the C-terminus of Aβ38 and the anti-Aβ42-antibody is directed to the C-terminus of Aβ42 (see also product information on G2-13, TGC, Zürich). Preferably, the antibodies bind to an epitope that is only present on the Aβ species to be detected.

The term "epitope" as used herein is meant to include any determinant responsible for specific interaction with an antibody molecule. Epitopic determinants usually consist of chemically active surface groupings of molecules such as amino acids or sugar side chains and have specific three-dimensional characteristics as well as specific charge characteristics.

The probes, e.g. an antibody, can be detectably labelled, for example, with a radioisotope, a bioluminescent compound, a chemiluminescent compound, a fluorescent compound, a metal chelate, or an enzyme (e.g. horse-radish peroxidase, alkaline phosphatase, β-galactosidase, malate dehydrogenase, glucose oxidase, urease, catalase etc.) which, in turn, when later exposed to a substrate will react to the substrate in such a manner as to produce a chemical moiety which can be detected. The probes can also be immobilized on an insoluble carrier, e.g. glass, polystyrene, polypropylene, polyethylene, dextran, nylon, natural and modified celluloses, polyacrylamides, agarose and magnetic beads.

Examples of immunoassays suitable for the method of the present invention are competitive or sandwich assays, such as the enzyme linked immunosorbent assay (ELISA), the radioimmunoassay (RIA) or Western Blots. Suitable antibody assay labels are known in the art and include enzyme labels, such as, glucose oxidase, and radioisotopes, such as iodine (¹²⁵I, ¹²¹I), carbon (¹⁴C), sulphur (³⁵S), tritium (³H), indium (¹¹²In), and technetium (⁹⁹mTc), and fluorescent labels, such as fluorescein and rhodamine.

In a further preferred embodiment, the method of the present invention is carried out in an ELISA format with a sandwich-ELISA format being even more preferred. Suitable ELISA and sandwich-ELISA formats are well known to the person skilled in the art.

The method of the present invention can be modified, e.g., by additionally determining the concentration of one or more further biomarkers (e.g., (i) the ratio of Aβ42:Aß40; (ii) the concentration of Aβ37 or Aß90; or (iii) the concentration of phosphor-tau (showing an increased concentration in AD patients) which might, in certain cases, further improve the diagnostic value of the method of the invention.

The method of the present invention for monitoring the progress of an AD therapy can be applied to any therapy, e.g., treatment with an NSAID (such as MPC-7869, R-flurbiprofen) or a statin.

For the diagnostic research discussed above, kits are useful. Preferably, such kits comprise an anti-Aβ38-antibody and an anti-Aβ42-antibody. The antibodies can be detectably labelled as described above and, preferably, allow diagnosis by ELISA. The kits might contain antibodies which are bound to a solid support, for example, a polystyrene microtiter dish or nitrocellulose paper. Alternatively, said kits are based on a RIA and contain said antibodies marked with a radioactive isotope. In a preferred embodiment of the kit of the invention the antibodies are labelled with enzymes, fluorescent compounds, luminescent compounds, ferromagnetic probes or radioactive compounds.

The following examples illustrate the invention.

### Example 1

### General Methods

### (A) Plasmids and transfections

Generation of ToxR-system plasmids has been described (Langosch et al., J Mol Biol 263 (1996), 525-30). APP695 with N-terminal Myc-tag and C-terminal Flag-tag or SPA4CT with C-terminal Flag-tag were used as a template to introduce G25A, G29A, G33A, G29/33A or G33I mutations by site-directed mutagenesis. The cDNAs were inserted into pCEP4 (Invitrogen, Karlsruhe, Germany), which contains a hygromycine-resistance gene. For stable expression, plasmids (2 µg) were transfected into SH-SY5Y cell line (ATCC number: CRL-2266) using Transfectine (Bio-Rad, München, Germany) following the manufacturer's instructions. For FRET-measurements, APP695 cDNAs were amplified with primers allowing in-frame ligation to CFP/YFP in vectors pcDNA3 (Invitrogen). All sequences were confirmed by dideoxy sequencing.

### (B) Fluorescence Resonance Energy Transfer (FRET)

For transient expression, plasmids (0.75 µg of pcDNA3-APP695-CFP and 1.5 µg of pcDNA3-APP695-YFP) were transfected into BEK293 cells (ATCC number: CRL1573) using the Fugene 6 transfection reagent (Roche Molecular Biochemicals, Mannheim, Germany) following the manufacturer's instructions. In competition FRET experiments, the amount of plasmid was reduced to 0.35 µg of pcDNA3-APP695-CFP and 0.75 µg of pcDNA3-APP695-YFP to allow addition of up to 1.5 µg of plasmid encoding competitor pCEP4-APP or pcDNA3. FRET measurements were performed as described by Voigt et al., J Biol Chem 280 (2005), 5121-7.

### (C) ToxR-system

E. coli FHK12 cells expressing chimera constructs were lysed and β-galactosidase activity was measured (Langosch et al., 1996). To control correct insertion of fusion proteins into the bacterial inner membrane, chimera were expressed in E. coli PD28 cells deficient for MBP (Brosig and Langosch, Protein Sci 7 (1998), 1052-6). Correct orientated chimera complement MBP deficiency when cells are grown in M9 minimal media with maltose as the only carbon source measured by cell densities after 48 h.

### (D) Sandwich ELISA, immunoprecipitation, and Western blots

Stably transfected cells were plated at a density of 2 x 10⁶ cells per 60-mm dish, The day after splitting, 4 ml of fresh media were added and incubated for 24 h. For Aβ40 and Aβ42-specific ELISAs, 50 µl of media were analyzed according to the manufacturer's instruction (The Genetics company (TGC), Basel, Switzerland). The same protocol was applied to determine Aβ38 levels, except that the antibody BAP-29 (provided by M. Brockhaus, Roche, Basel, Switzerland) was used. Aβ was precipitated from equal volumes of conditioned media with anti-serum (generated against residues 1-40). Aliquots of conditioned media were directly analyzed for secreted APP (sAPP). For full-length APP, cells were lysed in a buffer containing 50 mM Tris, pH 7.4, 150 mM NaCl, 2 mM EDTA, 2% NP40 and 2% Triton-X100. For detection of C-terminal fragments (β-CTF) of APP- and SPA4CT-transfected cells, cells were treated with γ-secretase inhibitor N-[N-(3,5-difluorophenacetyl-L-alanyl)]-S-phenylglycine t-butyl ester (DAPT) for 24 h, lysed, and equal amounts of protein were immunoprecipitated with an anti-serum (to APP cytosolic domain). Samples were separated by SDS-PAGE, transferred to nitrocellulose and immunolabelled either with antibody WO-2 to Aβ residues 2-10 or 879 (sAPPβ, provided by P. Paganetti, Novartis, Basel, Switzerland), or antibody 22C11 (Roche, Mannheim, Germany).

### (E) MALDI-MS

Aβ from conditioned media was immunoprecipitated with WO-2. Protein-G-Sepharose was washed in the presence of high salt (500 mM NaCl), followed by PBS and 100 mM ammonium chloride, pH 7.4. Aβ was eluted with 50% acetic acid and vacuum-dried. MALDI-MS analysis was carried out on sinapinic acid matrix with an UltraflexII TOF/TOF (Bruker Daltonics, Bremen, Germany.)

### (F) Molecular Modelling

For both helices five different, non-symmetrical starting conformations with identical backbone but varying random side chain conformations were generated. From each starting conformation, 65,000 structures were generated by randomly rotating the helix 0-360° around the long axis; translating it -2 to +6 Å toward or away from geometric centre, respectively, translating each helix ±10 Å along its long axis and tilting the helices relative to the diad axis by ±20°. A dielectric constant of 20 was used for the electrostatics and an energy map was created. The rotation was sampled in 36 degree bins, the tilt in 2.5 degree bins, the longitudinal shift in 2 Å bins and the translational shift was sampled in 0.8 Å bins. The average energy of each bin was evaluated independently by using Boltzmann averaging. The energy map was graphically evaluated, and a symmetric structure was assumed, so that energy minima corresponding to asymmetric structures were disregarded. A related procedure has been shown to reliably model helix bundles (Gottschalk, J Mol Graph Model 23 (2004), 99-110).

### Example 2

### Impact of the GxxxG motifs an full-length APP homodimerization

Biochemical studies had previously indicated the APP ectodomain in homodimerization by two different sites, one located at amino acids 448-465 that is overlapping with the collagen binding site and another at the loop region encompassing residues 91-111 (Beher et al., J Biol Chem 271 (1996), 1613-20; Scheuermann et al., J Biol Chem 276 (2001), 33923-9). The GxxxG motifs would therefore provide a third contact site of APP dimers. A fluorescence resonance energy transfer (FRET) approach was used to examine whether APP dimerization also depends on the GxxxG motif in living cells. Cyan or yellow fluorescent proteins (CFP or YFP) were fused to the C terminus of full-length APP695 wild-type (wt) or mutant constructs G33A, G33I or G29/33A, which are mutated within the central GxxxG motif. All fusion proteins showed identical subcellular distribution patterns in HEK293 cells as assessed by confocal microscopy (Figure 1B). For APPwt-YFP and APPwt-CFP, a FRET efficiency of 11.2% was determined, demonstrating for the first time close proximity of two APP molecules in living cells (Figure 1D). Since FRET signals were concentration-dependently reduced upon co-expression of non-fluorescent APPwt-Flag, it was concluded that the FRET signals observed are specific (Figure 1C). The specificity is further indicated by APP-YFP and the co-localization of the unrelated EGFR-CFP, a family member of the γ-secretase substrate ErbB-4, that does not show any detectable FRET efficiency, as expected (Figure 1C). The kinetics of FRET and regression analysis of acceptor photobleaching are shown in Figures 1E and 1F. To test the influence of the APP TMS GxxxG motifs an APP homodimerization, FRET efficiencies of APP G33A, APP G29/33A and APP G33I were measured. No major differences were observed for the mutants compared to the wt (Figure 1D). It is concluded that mutations of the GxxxG motif do not influence APP dimerization in general. Accordingly, GxxxG-mediated interactions seem to be secondary to the initiation of dimerization through the N-terminal contact sites (Beher et al., (1996); Scheuermann et al., (2001)) and might rather affect the TMS conformation within the membrane.

### Example 3

### The APP transmembrane sequence dimerizes via the GxxxG motifs

To selectively analyze the ability of the APP GxxxG motifs as part of the TMS to promote helix self-interaction, the ToxR-system (Langosh et al., (1996)) was applied, an assay to measure the homodimerization strength of a short TMS within the bacterial inner membrane. To this end, a fusion protein was constructed composed of the periplasmic maltose-binding protein (MBP), the TMS under study, which integrates into the bacterial inner membrane, and the cytosolic transcription activation domain ToxR (Figure 2A). TMS-driven dimerization within the membrane leads to close proximity of the ToxR-domains, which are able to activate transcription of the reporter gene encoding β-galactosidase only in the dimeric state. The dimerization strength of the TMS under study is thus reported by β-galactosidase activity. TMS Aβ residues 29 to 42 gave a strong signal similar to that obtained with residues 75 to 87 of glycophorin A (GpA) (Figure 2B), a well-investigated self-interacting. TMS (Langosch et al., (1996)). Dimerization strength was gradually reduced from Aβ TMS mutant constructs G29A and G33A to mutation G29/33A and was abolished by the mutation G33I (Figure 2B). Unfortunately, the influence of the juxtamembrane residue G25 cannot be measured by this system as only TMS sequences can be analyzed. Since the mutations G37A and G38A distal to the central GxxxG motif did not cause any effects, it is concluded that G29 and G33 are the key residues of the GxxxG motifs that determine the association of Aβ TMS. The differences between the double mutation G29/33A and mutation G33I might directly reflect the varying affinities of the two helices. While the mutation G29/33A might still allow the two TMS to approximate each other, stabilizing van der Waals bonding might occur. In contrast, the branched amino acid isoleucine of the G33I mutant might prevent this approximation and thus abolish dimerization. All ToxR-chimera inserted correctly into the bacterial membrane (Figure 2C) and showed equal expression levels (data not shown).

### Example 4

### GxxxG mutations reduce Aβ42 levels

Next it was examined whether the GxxxG mutations alter APP processing and generation of Aβ. SH-SY5Y cells stably expressing APP695wt or mutants G25A, G29A, G33A, G29/33A, or G33I were generated. For all cell lines, similar levels of full-length APP, soluble APP (sAPPα and sAPPβ), β-CTF and total secreted Aβ were observed by Western blot (Figure 3, A and C). Thus, GxxxG mutations neither affect shedding by α- or β-secretases nor interfere with maturation and surface expression of APP. However, when Aβ42, Aβ40 and Aβ38 species were quantified in a Sandwich enzyme linked immunosorbent assay (ELISA), significant differences to the wt were revealed. The mutants G25A, G29A and G33A showed no alterations in Aβ40 levels, but Aβ42 secretion was significantly reduced by 28%, 67%, and 60%, respectively, compared to cell lines expressing APPwt (Figure 4, A and C). Thus, G29A and G33A reduced Aβ42 levels to a similar extent, although G33A attenuated dimerization of the TMS more efficiently than G29A (Figure 2B). This might be due to the bacterial membrane insertion of Aβ residues 29 to 42 with G29 located at the boundary of the chimera constructs used in the ToxR-assay (Figure 2B). In addition, residue G25 at the extracellular juxtamembrane position appears to be an extended part of the GxxxG dimerization motif since the G25A mutant reduced Aβ42 levels similar to mutations G29A and G33A. The double mutation G29/33A slightly reduced Aβ40 generation and decreased the Aβ42 level to the background secretion of mock-transfected cells (Figure 4, A and C). Since the mutation G33I abolished the generation of Aβ40 and Aβ42 but no reduction of total Aβ was observed by Western blot (Figure 3A) it was assumed that this mutant might lead to the secretion of other Aβ species (see Example 6).

### Example 5

### Effects of GxxxG mutants are independent of the APP ectodomain

To exclude influences of the APP ectodomain contact sites or β-secretase activity an Aβ generation of GxxxG mutants, secreted Aβ of SH-SY5Y cells stably transfected with the construct SPA4CT (Dyrks et al., FEBS Lett 309 (1992), 20-24) were analyzed, which releases β-CTF after cleavage of the signal peptide (SP) independently from β-secretase (Figure 3C). SPA4CTwt- or mutant transfected cells showed equal expression levels of β-CTF and Aβ by Western blot analysis (Fig. 3B) . Generation of Aβ40 and Aβ42 of SPA4CTwt and mutants yielded similar levels of Aβ as gained from the corresponding full-length APP constructs, described above (Figure 4, B and D). This indicates that the effect of the GxxxG motif an Aβ production is independent from the APP ectodomain.

### Example 6

### Aβ38 is produced at the expense of Aβ42

Since total Aβ levels from APPwt and G33I and G29/33A mutants appeared similar as observed by Western blot (Figure 3A), samples were analyzed for the presence of C-terminally truncated Aβ species by MALDI-MS. In the culture supernatant from APP G33I-expressing cells Aβ38 and Aβ37 gave the most prominent signals whereas Aβ40 could hardly be detected (Figure 4E). Aβ from APP G29/33A-expressing cells also showed a much stronger signal for Aβ38 (data not shown). To investigate if the seemingly increasing amounts of Aβ38 (Figure 4E) are produced at the expense of Aβ42, the level of Aβ38 secreted from β-CTF-expressing cells was determined by using an Aβ38 specific ELISA (Figure 4F). The mutations G29/33A and G33I showed the highest amount of Aβ38 produced (mutation G29/33A was set as 100%) corresponding to their abolished Aβ42 generation and reduced Aβ40 levels. Compared to the double mutation G29/33A the single substitutions G29A and G33A generated less Aβ38 (36% and 52%, respectively) as they specifically reduced Aβ42 generation but not Aβ40. The mutation G25A showed no significant change in Aβ38 levels compared to the wt. The mutant G33I led to slightly less Aβ38 produced compared to mutant G29/33A but instead led to increased amounts of Aβ37 (Figure 4E). Thus, there is a strong inverse correlation between the generation of Aβ42 and Aβ38 depending on the GxxxG mutations. The gradually decreasing or increasing levels of Aβ42 and Aβ38 clearly depend on the individual glycine substitutions and are either attributed to conservative or non-conservative mutations in their consequence. The data indicates that the Aβ42 and A38 production is intimately linked to the dimerization strength of the TMS-TMS interaction through the residues G29 and G33 of the GxxxG motifs as indicated by the ToxR-assay. The results show that mutations of the GxxxG motifs do not affect cleavage efficiency but shift the γ-secretase cleavage to more N-terminal sites.

### Example 7

### A helix-helix interface with G29 and G33 is favoured conformationally

In order to test whether a helix-helix interaction through the GxxxG motif is favoured energetically and conformationally, a computational search of conformations of Aβ residues 25 to 46 was performed. A low-energy conformation with a right-handed crossing angle and all four glycines of the three consecutive GxxxG motifs at the helix-helix interface was observed (Figure 5A). According to this model, residues G29 and G33 constitute the core of the interface with Cα-Cα distances of less than 5 Å for each pair compared to the distances of G25 (6.7 Å) and G37 (5.7 Å).

## Claims

1. A method for the diagnosis of Alzheimer's Disease (AD) or the stage of AD, said method comprising the following steps:
(a) contacting a sample obtained from a patient with probes specifically binding to at least two y-secretase cleavage products Aß48, Aß45, Aß42, Aß38 and Aß35,; and
(b) determining the amounts of said cleavage products in said sample, wherein at least the Aß38:Aß42 ratio is determined and a decreased ratio of Aß38:Aß42 as compared to the normal ratio is indicative for AD.

2. A method for monitoring the progress of an AD therapy, said method comprising the following steps:
(a) contacting a sample obtained from a patient with probes specifically binding to at least two y-secretase cleavage products Aß48, Aß45, Aß42, Aß38 and Aß35 ; and
(b) determining the amounts of said cleavage products in said sample, wherein a normalized ratio of said cleavage products as compared to the ratio at the onset of therapy is indicative of a therapeutic effect.

3. A diagnostic method for distinguishing between Alzheimer's Disease (AD) and non-AD, said method comprising the following steps:
(a) contacting a sample obtained from a patient with probes specifically binding to the y-secretase cleavage products Aß42, Aß40 and Aß38; and
(b) determining the amounts of said cleavage products in said sample,
wherein a ratio of Aß38:Aß42 > 1 in combination with a normal ratio of Aß42:Aß40 is indicative of non-AD.

4. The method of claim 2, wherein the ratio of Aß38:Aß42 is determined.

5. The method of claim 1, wherein (i) a decreased ratio of Aß38:Aß42 as compared to the normal ratio and an increased concentration of Aß42 as compared to the normal concentration is indicative of an early stage of AD and (ii) a decreased ratio of Aß38:Aß42 as compared to the normal ratio and a decreased or normal concentration of Aß42 as compared to the normal concentration is indicative of a late stage of AD.

6. The method of claim 4 or 5, wherein the ratio of Aß38:Aß42 is ≤ 1.5.

7. The method of any one of claims 1 to 6, wherein the sample is CSF or blood.

8. The method of any one of claims 1 to 6, wherein the probes are antibodies.

9. The method of claim 8, wherein the antibodies are monoclonal antibodies.

10. The method of claim 9, wherein the method is carried out as ELISA.

11. The method of claim 10, wherein the ELISA is a sandwich-ELISA.

12. The method of any one of claims 1 to 11, comprising the determination of the concentration of at least one additional biomarker comprising (i) the ratio of Aß42:Aß40, (ii) the concentration of Aß37 or Aß40; or (iii) the concentration of phospho-tau.

13. The method of claim 2, wherein therapy comprises treatment with a non-steroidal anti-inflammatory drug (NSAID) or statin.

14. The method of claim 13, wherein the non-steroidal anti-inflammatory drug (NSAID) is MPC-7869 (R-flurbiprofen).

## Patentansprüche

1. Verfahren zur Diagnose von Alzheimer (AD) oder einem Stadium von Alzheimer, wobei das Verfahren die folgenden Schritte umfasst:
(a) Kontaktieren einer Probe, die von einem Patienten erhalten wird, mit Sonden, die spezifisch an mindestens zwei γ-Sekretase-Spaltprodukte Aß48, Aß45, Aß42, Aß38 und Aß35 binden; und
(b) Bestimmen der Mengen der Spaltprodukte in der Probe, wobei zumindest das Verhältnis Aß38:Aß42 bestimmt wird und ein reduziertes Verhältnis von Aß38:Aß42 im Vergleich zum normalen Verhältnis ein Hinweis auf Alzheimer ist.

2. Verfahren zum Überwachen des Fortschritts einer Alzheimer-Therapie, wobei das Verfahren die folgenden Schritte umfasst:
(a) Kontaktieren einer Probe, die von einem Patienten erhalten wird, mit Sonden, die spezifisch an mindestens zwei y-Sekretase-Spaltprodukte Aß48, Aß45, Aß42, Aß38 und Aß35 binden; und
(b) Bestimmen der Mengen der Spaltprodukte in der Probe, wobei ein normalisiertes Verhältnis der Spaltprodukte im Vergleich zum Verhältnis zu Beginn der Therapie ein Hinweis auf eine therapeutische Wirkung ist.

3. Diagnostisches Verfahren zum Differenzieren zwischen Alzheimer (AD) und Nicht-AD, wobei das Verfahren die folgenden Schritte umfasst:
(a) Kontaktieren einer Probe, die von einem Patienten erhalten wird, mit Sonden, die spezifisch an die y-Sekretase-Spaltprodukte Aß42, Aß40 und Aß38 binden; und
(b) Bestimmen der Mengen an Spaltprodukten in der Probe,
wobei das Verhältnis von Aß38:Aß42 > 1 in Kombination mit einem normalen Verhältnis von Aß42:Aß40 ein Hinweis auf Nicht-AD ist.

4. Verfahren nach Anspruch 2, wobei das Verhältnis von Aß38:Aß42 bestimmt wird.

5. Verfahren nach Anspruch 1, wobei (i) ein reduziertes Verhältnis von Aß38:Aß42 im Vergleich zum normalen Verhältnis und eine erhöhte Konzentration von Aß42 im Vergleich zur normalen Konzentration ein Hinweis auf ein frühes Stadium von Alzheimer ist und (ii) ein reduziertes Verhältnis von Aß38:Aß42 im Vergleich zum normalen Verhältnis und eine reduzierte oder normale Konzentration von Aß42 im Vergleich zur normalen Konzentration ein Hinweis auf ein spätes Stadium von Alzheimer ist.

6. Verfahren nach Anspruch 4 oder 5, wobei das Verhältnis von Aß38:Aß42 ≤ 1,5 ist.

7. Verfahren nach einem der Ansprüche 1 bis 6, wobei die Probe CSF oder Blut ist.

8. Verfahren nach einem der Ansprüche 1 bis 6, wobei die Sonden Antikörper sind.

9. Verfahren nach Anspruch 8, wobei die Antikörper monoklonale Antikörper sind.

10. Verfahren nach Anspruch 9, wobei das Verfahren als ELISA durchgeführt wird.

11. Verfahren nach Anspruch 10, wobei der ELISA ein Sandwich-ELISA ist.

12. Verfahren nach einem der Ansprüche 1 bis 11, umfassend die Bestimmung der Konzentration von mindestens einem zusätzlichen Biomarker, umfassend (i) das Verhältnis Aß42:Aß40, (ii) die Konzentration von Aß37 oder Aß40; oder (iii) die Konzentration von phospho-tau.

13. Verfahren nach Anspruch 2, wobei die Therapie die Behandlung mit einem nicht steroidalen, entzündungshemmenden Arzneimittel (NSAID) oder Statin umfasst.

14. Verfahren nach Anspruch 13, wobei das nicht steroidale, entzündungshemmende Arzneimittel (NSAID) MPC-7869 (R-Flurbiprofen) ist.

## Revendications

1. Procédé pour le diagnostic d'une maladie d'Alzheimer (AD) ou du stade d'une AD, ledit procédé comprenant les étapes suivantes :
a) mettre en contact un échantillon obtenu à partir d'un patient avec des sondes se liant spécifiquement à au moins deux produits de division de secrétase-γ Aβ48, Aβ45, Aβ42, Aβ38 et Aβ35 ; et
b) déterminer les quantités desdits produits de division dans ledit échantillon, pour lequel au moins le rapport Aβ38 : Aβ42 est déterminé, et un rapport réduit de Aβ38 : Aβ42, en comparaison avec le rapport normal, est indicatif d'une AD.

2. Procédé pour surveiller les progrès d'une thérapie contre l'AD, ledit procédé comprenant les étapes suivantes :
a) mettre en contact un échantillon obtenu à partir d'un patient avec des sondes se liant spécifiquement à au moins deux produits de division de secrétase-γ Aβ48, Aβ45, Aβ42, Aβ38 et Aβ35 ; et
b) déterminer les quantités desdits produits de division dans ledit échantillon, pour lequel un rapport normalisé desdits produits de division, en comparaison avec le rapport du début de la thérapie, est indicatif d'un effet thérapeutique.

3. Procédé de diagnostic pour effectuer une distinction entre une maladie d'Alzheimer (AD) et non une AD, ledit procédé comprenant les étapes suivantes :
a) mettre en contact un échantillon obtenu à partir d'un patient avec des sondes se liant spécifiquement aux produits de division de secrétase-γ Aβ42, Aβ40 et Aβ38 ; et
b) déterminer les quantités desdits produits de division dans ledit échantillon, pour lequel un rapport de Aβ38 : Aβ42 > 1, en combinaison avec un rapport normal de Aβ42 : Aβ40, est indicatif de non une AD.

4. Procédé selon la revendication 2, pour lequel le rapport de Aβ38 : Aβ42 est déterminé.

5. Procédé selon la revendication 1, pour lequel i) un rapport réduit de Aβ38 : Aβ42, en comparaison avec le rapport normal, et une concentration augmentée de Aβ42, en comparaison avec la concentration normale, sont indicatifs d'un stade précoce d'une AD et ii) un rapport réduit de Aβ38 : Aβ42, en comparaison avec le rapport normal, et une concentration réduite ou normale de Aβ42, en comparaison avec la concentration normale, sont indicatifs d'un stade tardif d'une AD.

6. Procédé selon la revendication 4 ou 5, pour lequel le rapport de Aβ38 : Aβ42 est ≤ 1,5.

7. Procédé selon l'une quelconque des revendications 1 à 6, pour lequel l'échantillon est du liquide céphalorachidien ("CSF") ou du sang.

8. Procédé selon l'une quelconque des revendications 1 à 6, pour lequel les sondes sont des anticorps.

9. Procédé selon la revendication 8, pour lequel les anticorps sont des anticorps monoclonaux.

10. Procédé selon la revendication 9, pour lequel le procédé est mis en oeuvre en tant qu'ELISA.

11. Procédé selon la revendication 10, pour lequel l'ELISA est une ELISA en sandwich.

12. Procédé selon l'une quelconque des revendications 1 à 11, comprenant la détermination de la concentration d'au moins un bio-marqueur additionnel comprenant i) le rapport de Aβ42 : Aβ40 ; ii) la concentration de Aβ37 ou de Aβ40 ; ou iii) la concentration de phospho-tau.

13. Procédé selon la revendication 2, pour lequel une thérapie comprend un traitement avec un médicament anti-inflammatoire non-stéroïdien ("NSAID") ou une statine.

14. Procédé selon la revendication 13, pour lequel le médicament anti-inflammatoire non-stéroïdien ("NSAID") est du MPC-7869 (R-flurbiprofène).
